# EUROPEAN PATENT APPLICATION

(11) **EP 4 737 902 A1**
(43) Date of publication of application: **06.05.2026**
(21) Application number: 24832474.1
(22) Date of filing: 27.06.2024
(51) Int. Cl.: G01N 33/68, C12Q 1/6883, G01N 33/50

(54) **COMPOSITION FOR DIAGNOSING PARKINSON?S DISEASE AND METHOD FOR DIAGNOSING PARKINSON?S DISEASE BY USING SAME**

(30) Priority: 27.06.2023 KR 20230083059
(71) Applicant: Ninebiowear Co., Ltd., Suwon-si, Gyeonggi-do 16419 (KR)
(72) Inventor: LEE, Yunjong, Suwon-si Gyeonggi-do 16365 (KR); LEE, Nae-Eung, Seoul 04036 (KR); SHIN, Jeong Yong, Gwangju 61175 (KR); KIM, Heejeong, Suwon-si Gyeonggi-do 16357 (KR); CHO, Seok Hyun, Seoul 05276 (KR); KIM, Hee Tae, Seoul 04164 (KR)
(74) Representative: Herrero & Asociados, S.L.
(86) International application number: PCT/KR2024/009023
(87) International publication number: WO 2025/005697

(57) **Abstract**

The present invention relates to a composition for diagnosing Parkinson's disease, comprising an agent for detecting a protein consisting of a combination of GLUT3 and any one or more selected from the group consisting of USP14, α-synuclein, and AIMP2, or a gene encoding the protein. The composition for diagnosing Parkinson's disease and a diagnostic kit comprising the composition, of the present invention, can be used to perform detection or diagnosis by distinguishing Parkinson's disease patient groups through a simple blood test. Furthermore, by primarily applying same to patients showing prodromal symptoms of Parkinson's disease, such as olfactory dysfunction, sleep disorders, and constipation, the composition and the diagnostic kit can be used as a preliminary test to determine whether to perform neurological examinations and brain imaging tests at large hospitals. Also, the composition and the diagnostic kit can be used as a means for quantifying the effect of treating Parkinson's disease.

## Description

### Technical Field

The present disclosure was carried out with the support of the Ministry of Science and ICT of the Republic of Korea under Project No. 2021M3H4A4079521, wherein the research management agency is the National Research Foundation of Korea, the project title is "Development of Nanomaterials Technology," the research subject is "Development of Isothermal Amplification Electrochemical Molecular and Digital Fluorescence Imaging Immunodiagnostic Device," the supervising institution is Sungkyunkwan University, and the research period is from February 1, 2022 to January 31, 2023.

The present disclosure was carried out with the support of the Ministry of Health and Welfare of the Republic of Korea under Project No. HU22C0143000022, wherein the research management agency is the Korea Health Industry Development Institute, the project title is "Dementia Overcoming Research and Development Project (Ministry of Health and Welfare)," the research subject is "Elucidation of the Pathogenesis and Lesion Propagation Brain Map of Lewy Body Dementia Based on the Alpha-Synuclein Aggregation/Propagation Promoting System Targeting AIMP2," the supervising institution is the Sungkyunkwan University Research & Business Foundation, and the research period is from April 1, 2022 to December 28, 2022.

The present application claims the benefit of and priority to Korean Patent Application No. 10-2023-0083059, filed with the Korean Intellectual Property Office on June 27, 2023, the disclosure of which is incorporated herein by reference.

The present disclosure relates to a composition for diagnosing Parkinson's disease through detection of expression level of at least one protein selected from the group consisting of GLUT3, USP14, α-synuclein, and AIMP2 in blood, or a gene coding therefor.

### Background Art

Parkinson's disease is a neurodegenerative disorder, in which dopaminergic neurons of the substantia nigra located in the midbrain are destroyed, thereby causing movement disorders. Patients with Parkinson's disease exhibit not only motor symptoms but also non-motor symptoms such as olfactory dysfunction, sleep disorders, depression, mania, and cognitive impairment. In modern society, which is rapidly entering an aging era, the number of patients with Parkinson's disease is also rapidly increasing. According to the disease statistics data of the National Health Insurance Service, the number of Parkinson's disease patients in Korea increased approximately 2.5-fold, from 39,265 in 2004 to 96,499 in 2016.

Diagnosis of Parkinson's disease relies heavily on quantitative scoring of motor/non-motor symptoms associated with Parkinson's disease based on the Unified Parkinson's Disease Rating Scale(UPDRS) by specialists. However, it has been reported that the misdiagnosis rate reaches as high as 25% in the early stage of Parkinson's disease (Thomas G. Beach et al., 2018). Although the loss of dopaminergic neurons can be diagnosed early by capturing DaTscan brain imaging of the axons of dopaminergic neurons, accessibility to patients is limited since such diagnosis can only be performed in large hospitals equipped with expensive equipment.

For accurate and accessible diagnosis of Parkinson's disease, it is important to develop molecular diagnostic markers and portable diagnostic devices utilizing same. Molecular diagnostic markers can be used not only for diagnosis of the disease but also for quantification of symptom alleviation in clinical trials and prediction of treatment prognosis, thereby highlighting the need for research in this field.

Throughout the specification, numerous papers and patent documents are referred to and cited. The disclosure of the cited papers and patent documents are incorporated herein in their entirety by reference, thereby clarifying the level of ordinary skill in the art to which the present disclosure pertains and enabling a clearer understanding of the present disclosure.

### Disclosure of Invention

### Technical Problem

Leading to the present disclosure, intensive and thorough research conducted by the present inventors, with the aim of developing a diagnostic method that is capable of determining the possibility of onset of Parkinson's disease and enabling early diagnosis and which allows simple diagnosis of Parkinson's disease in a minimally invasive manner, resulted in the finding that early diagnosis of Parkinson's disease is possible through detection of the expression levels of GLUT3 and at least one protein selected from the group consisting of USP14, α-synuclein, and AIMP2 in blood.

Accordingly, an aspect of the present disclosure is to provide a composition for diagnosing Parkinson's disease.

Another aspect of the present disclosure is to provide a kit for diagnosing Parkinson's disease.

Still another aspect of the present disclosure is to provide a method for providing information for diagnosing Parkinson's disease.

A further aspect of the present disclosure is to provide a method for screening a therapeutic agent for Parkinson's disease.

Other objectives and advantages of the present disclosure will be more clearly understood from the following detailed description of the invention, the claims, and the drawings.

### Solution to Problem

Provided according to one aspect of the present disclosure is provided a composition for diagnosing Parkinson's disease, the composition further comprising an agent for detecting at least one protein selected from the group consisting of glucose transporter 3(GLUT3), ubiquitin-specific protease 14(USP14), α-synuclein, and aminoacyl-tRNA synthetase complex interacting multifunctional protein-2(AIMP2), aggregates thereof, or genes encoding same.

Another aspect of the present disclosure provides a composition for diagnosing Parkinson's disease, comprising a glucose transporter 3(GLUT3) protein, aggregates thereof, or an agent for detecting genes encoding same.

The present inventors made intensive research efforts to identify a diagnostic method capable of determining the possibility of onset of Parkinson's disease and enabling early diagnosis in a minimally invasive and simple manner. As a result, the inventors identified that early diagnosis of Parkinson's disease is possible through detection of proteins comprising GLUT3 in blood plus at least one protein selected from the group consisting of USP14, α-synuclein, and AIMP2 or a gene encoding same in blood.

As used herein, the term "aggregate" is intended to encompass polymers of proteins, fibrils, fibril precursors, plaques, protein monomers and polymers bound to other proteins, proteins bound to lipids or carbohydrates, proteins bound to nucleic acids, and degraded forms of protein-bound blood cells, wherein the fibrils may include oligomers of abnormal proteins.

In addition, the aggregates may be those generated by aggregation of intrinsically disordered proteins(IDPs) or misfolded proteins caused by various factors such as DNA sequence mutations, problems in the transcription/translation stage of DNA, temperature, pH, oxidative stress, environmental stress, aging, etc.

By using the composition of the present disclosure, patients with Parkinson's disease can be discriminated and diagnosed through a simple blood test. Furthermore, the composition can be applied preferentially to patients showing prodromal symptoms of Parkinson's disease, such as olfactory dysfunction, sleep disorders, and constipation, and can thus be utilized as a preliminary test to determine whether to proceed with neurological examinations or brain imaging in large hospitals. In addition, it can also be utilized as a means for quantifying the therapeutic effect of Parkinson's disease treatment.

As used herein, the term "diagnosis" means determining the susceptibility of a subject to a specific disease or disorder, or determining whether the subject currently has a specific disease or disorder.

The composition for diagnosing Parkinson's disease of the present disclosure may comprise at least one protein selected from the group consisting of GLUT3, USP14, α-synuclein, and AIMP2, an aggregate thereof, or an agent for detecting a gene encoding same.

The agent for detecting GLUT3, USP14, α-synuclein, and AIMP2 proteins may detect not only the normal structure of the proteins but also aggregated forms (oligomers, amyloid aggregates) appearing in a disease state.

Unless otherwise specified, the agent for detecting at least one protein selected from the group consisting of GLUT3, USP14, α-synuclein, and AIMP2 may be used for quantification and analysis of both the normal structure and denatured aggregate structure of the protein.

However, a method for separately measuring the normal and misfolded structures of the four proteins may also be applied and utilized in diagnosis.

When used, the composition for diagnosing Parkinson's disease, comprising an agent for detecting GLUT3 protein of the present disclosure, allows for diagnosis of Parkinson's disease with high sensitivity and specificity.

When using the composition for diagnosing Parkinson's disease comprising an agent for detecting GLUT3, USP14, α-synuclein, and AIMP2 proteins of the present disclosure, Parkinson's disease can be diagnosed with remarkably high sensitivity and specificity. This means that although Parkinson's disease can be diagnosed using only an agent for detecting GLUT3 protein in plasma, diagnostic accuracy is significantly improved when used in combination with agents for detecting USP14, α-synuclein, and AIMP2 proteins.

The at least one protein selected from the group consisting of GLUT3, USP14, α-synuclein, and AIMP2, or the gene encoding same, of the present disclosure may be present in a biological sample isolated from a subject, and more specifically, for example, in blood, serum, or plasma.

In an embodiment of the present disclosure, the at least one protein selected from the group consisting of GLUT3, USP14, α-synuclein, and AIMP2 refers to a protein present in a blood sample isolated from a human body.

Conventionally known methods for diagnosing Parkinson's disease are quantitative scoring methods of Parkinson's disease-associated motor and non-motor symptoms based on the Unified Parkinson's Disease Rating Scale(UPDRS) by specialists. However, since it has been reported that the misdiagnosis rate in the early stage of Parkinson's disease reaches 25% (Thomas G. Beach et al., 2018), there are limitations in applying such methods to early diagnosis. Although the loss of dopaminergic neurons can be diagnosed early by DaTscan brain imaging of the axons of dopaminergic neurons, accessibility is limited to large hospitals equipped with expensive imaging devices.

In contrast, when using the composition for diagnosing Parkinson's disease according to one embodiment of the present disclosure, Parkinson's disease can be rapidly, simply, and accurately diagnosed at an early stage by measuring the expression level of at least one protein selected from the group consisting of GLUT3, USP14, α-synuclein, and AIMP2, or a gene encoding same, in blood, serum, or plasma isolated from a subject.

In an embodiment of the present disclosure, the agent for detecting proteins of the present disclosure is one or more selected from the group consisting of an antibody, an aptamer, a ligand, a peptide nucleic acid(PNA), and an oligopeptide.

As used herein, the term "detecting proteins" refers to the process of confirming the presence and expression level of GLUT3, USP14, α-synuclein, or AIMP2 proteins, which are diagnostic markers for Parkinson's disease in the present disclosure, in a biological sample such as blood, for the purpose of diagnosing Parkinson's disease. Methods for measuring expression levels of the proteins or comparative analysis include, but are not limited to, protein chip analysis, immunoassays, ligand binding assays, Matrix Assisted Laser Desorption/lonization Time of Flight Mass Spectrometry(MALDI-TOF) analysis, Surface Enhanced Laser Desorption/Ionization Time of Flight Mass Spectrometry(SELDI-TOF) analysis, radioimmunoassay, radial immunodiffusion, Ouchterlony immunodiffusion, rocket immunoelectrophoresis, immunohistochemistry, complement fixation assay, two-dimensional electrophoresis analysis, liquid chromatography-mass spectrometry(LC-MS), liquid chromatography-tandem mass spectrometry(LC-MS/MS), Western blotting, dot blotting, and enzyme-linked immunosorbent assay(ELISA).

As used herein, the term "antibody" refers to a substance that specifically binds to an antigen to elicit an antigen-antibody reaction. For the purposes of the present disclosure, the antibody means an antibody that specifically binds to GLUT3, USP14, α-synuclein, or AIMP2 proteins. The antibodies of the present disclosure are intended to encompass polyclonal antibodies, monoclonal antibodies, and recombinant antibodies. The antibodies may be readily produced using techniques well known in the art. For example, a polyclonal antibody may be produced by a well-known method involving injecting the GLUT3, USP14, α-synuclein, or AIMP2 protein antigen into an animal, followed by collecting blood from the animal to obtain serum containing the antibody. Such polyclonal antibodies may be produced from any animal, such as goats, rabbits, sheep, monkeys, horses, pigs, cattle, or dogs. Further, monoclonal antibodies may be prepared by hybridoma methods(see Kohler and Milstein, European Journal of Immunology 6:511-519, 1976) or phage antibody library technologies(see Clackson et al., Nature, 352:624-628, 1991; Marks et al., J. Mol. Biol., 222:581-597, 1991), both of which are well known in the art. The antibodies prepared by the above methods may be isolated and purified by methods such as gel electrophoresis, dialysis, salt precipitation, ion-exchange chromatography, and affinity chromatography. The antibodies of the present disclosure include not only complete forms having two intact light chains and two intact heavy chains, but also functional fragments of antibody molecules. Functional fragments of an antibody molecule refer to fragments that retain at least antigen-binding activity, such as Fab, F(ab'), F(ab')2, and Fv.

The term "aptamer," as used herein, refers to an oligonucleotide or peptide molecule, and with regard to the general and detailed knowledge of aptamers, reference may be made to the literature[Bock LC et al., Nature 355(6360):5646 (1992); Hoppe-Seyler F, Butz K, "Peptide aptamers: powerful new tools for molecular medicine," J Mol Med. 78(8):426-430 (2000); Cohen BA, Colas P, Brent R., "An artificial cell-cycle inhibitor isolated from a combinatorial library," Proc. Natl. Acad. Sci. USA. 95(24):14272-14277 (1998)].

The term "ligand," as used herein, refers to a low molecular weight compound capable of binding to GLUT3, USP14, α-synuclein, or AIMP2 proteins to form a complex and generate a signal. The term may also be broadly construed to encompass antibodies.

As used herein, the term "peptide nucleic acid" (PNA) refers to an artificially synthesized polymer similar to DNA or RNA, first introduced in 1991 by Professors Nielsen, Egholm, Berg, and Buchardt of the University of Copenhagen, Denmark. Unlike DNA, which has a phosphate-ribose sugar backbone, PNA has a repeated N-(2-aminoethyl)-glycine backbone connected by peptide bonds. As a result, PNA has greatly increased binding affinity and stability to DNA or RNA, and has been applied in molecular biology, diagnostic analysis, and antisense therapeutics. PNA is disclosed in detail in [P. E. Nielsen, M. Egholm, R. H. Berg, O. Buchardt, "Sequenceselective recognition of DNA by strand displacement with a thyminesubstituted polyamide," Science 254(5037):1497-1500 (1991)].

In an embodiment of the present disclosure, the agent for detecting genes is one or more selected from the group consisting of primers, probes, and antisense nucleotides. Since information on GLUT3, USP14, α-synuclein, or AIMP2 proteins according to the present disclosure is already known, those skilled in the art can readily design primers, probes, or antisense nucleotides that specifically bind to mRNA encoding the proteins.

As used herein, the term "detecting genes" refers to the process of confirming the presence and expression levels of mRNAs encoding GLUT3, USP14, α-synuclein, or AIMP2 proteins in a biological sample for the diagnosis of Parkinson's disease, and measuring the amount of mRNA. Analysis methods therefor include, but are not limited to, reverse transcription polymerase chain reaction(RT-PCR), competitive reverse transcription PCR(Competitive RT-PCR), real-time reverse transcription PCR(Real-time RT-PCR), RNase protection assay(RPA), Northern blotting, and DNA chips.

The term "primer," as used herein, refers to a fragment that recognizes a target gene sequence and includes a pair of forward and reverse primers, preferably a pair of primers providing specific and sensitive analytical results. When the nucleotide sequence of a primer is mismatched with non-target sequences present in a sample so that only target gene sequences containing complementary primer-binding sites are amplified without inducing non-specific amplification, high specificity can be achieved.

As used herein, the term "probe" refers to a substance capable of specifically binding to a target substance to be detected in a sample, and refers to a substance that can confirm the presence of the target substance in the sample through the binding. The type of probe is not particularly limited so long as it is a substance conventionally used in the art, but is preferably a peptide nucleic acid(PNA), locked nucleic acid(LNA), peptide, polypeptide, protein, RNA, or DNA. More specifically, the probe may be a biomaterial derived from or similar to a biological source, or produced ex vivo, for example, enzymes, proteins, antibodies, microorganisms, animal or plant cells and tissues, nerve cells, DNA, and RNA. The DNA may include cDNA, genomic DNA, and oligonucleotides, and the RNA may include genomic RNA, mRNA, and oligonucleotides. Examples of proteins include antibodies, antigens, enzymes, and peptides.

The term "locked nucleic acid"(LNA), as used herein, refers to a nucleic acid analog containing a 2'-O, 4'-C methylene bridge [J. Weiler, J. Hunziker, and J. Hall, Gene Therapy (2006) 13, 496-502]. LNA nucleosides include common nucleic acid bases of DNA and RNA and are capable of forming base pairs according to Watson-Crick base pairing rules. However, due to the "locking" of the molecule by the methylene bridge, LNAs are unable to form an ideal conformation in Watson-Crick pairing. When included in DNA or RNA oligonucleotides, LNAs can pair more rapidly with complementary nucleotide strands and increase the stability of the double helix.

The term "antisense," as used herein, refers to an oligonucleotide having a nucleotide sequence and inter-subunit backbone that allows hybridization of an antisense oligomer with a target sequence in RNA through Watson-Crick base pairing, thereby typically allowing the formation of an RNA:oligomer heteroduplex within the target sequence. The oligomer may have exact or approximate sequence complementarity to the target sequence.

Another aspect of the present disclosure provides a kit for diagnosing Parkinson's disease, comprising the composition for diagnosing Parkinson's disease according to another aspect of the present disclosure described above.

As used herein, the term "kit" may refer to an RT-PCR kit, a DNA chip kit, an ELISA kit, a protein chip kit, a rapid kit, or a multiple reaction monitoring(MRM) kit, but with no limitations thereto.

The kit for diagnosing Parkinson's disease of the present disclosure may further include one or more additional compositions, solutions, or devices suitable for the analytical method. For example, the diagnostic kit may further include essential elements necessary for performing reverse transcription polymerase chain reaction(RT-PCR). The RT-PCR kit includes a primer pair specific for a gene encoding the marker protein. The primers are nucleotides having sequences specific to the nucleic acid sequence of the gene and may have a length of about 7 bp to 50 bp, preferably about 10 bp to 30 bp. The RT-PCR kit may also include primers specific for the nucleic acid sequence of a control gene. In addition, the RT-PCR kit may include test tubes or other suitable containers, reaction buffers (with varying pH and magnesium concentrations), deoxynucleotides(dNTPs), enzymes such as Taq polymerase and reverse transcriptase, DNase, RNase inhibitors, DEPC-treated water, and sterilized water.

In addition, the diagnostic kit of the present disclosure may include essential elements necessary for performing DNA chip analysis. The DNA chip kit may include a substrate to which cDNA or oligonucleotides corresponding to a gene or fragments thereof are attached, and reagents, preparations, or enzymes for producing fluorescently labeled probes. The substrate may further include cDNA or oligonucleotides corresponding to a control gene or fragments thereof.

Furthermore, the diagnostic kit of the present disclosure may include essential elements necessary for performing ELISA. The ELISA kit includes an antibody specific for the protein. The antibody is a monoclonal antibody, polyclonal antibody, or recombinant antibody, which has high specificity and affinity for the marker protein and little to no cross-reactivity with other proteins. The ELISA kit may also include an antibody specific for a control protein. In addition, the ELISA kit may include reagents for detecting bound antibodies, such as labeled secondary antibodies, chromophores, enzymes(e.g., conjugated with antibodies), substrates thereof, or other substances capable of binding to the antibodies.

The "kit for diagnosing Parkinson's disease" of the present disclosure includes the "composition for diagnosing Parkinson's disease" as described in another aspect of the present disclosure, and overlapping content is incorporated by reference, with repetitive description omitted in order to avoid excessive complexity of the present specification.

Provided according to another aspect of the present disclosure is a method for providing information for diagnosing Parkinson's disease, the method comprising the steps of:
(a) measuring an expression level of GLUT3 protein, aggregates thereof, or a gene encoding same in a blood sample isolated from a human; and
(b) providing information that the likelihood of Parkinson's disease is high when the expression level is increased compared to a normal blood control sample.

In the method for providing information for diagnosing Parkinson's disease according to an embodiment of the present disclosure, step (a) includes the following step (a-1) below and step (b) includes the following step (b-1):
(a-1) measuring an expression level of GLUT3 and at least one protein selected from the group consisting of USP14, α-synuclein, and AIMP2, an aggregate thereof, or a gene encoding same; and
(b-1) providing information that the likelihood of Parkinson's disease is high when the expression level of GLUT3 and at least one protein selected from the group consisting of USP14, α-synuclein, and AIMP2, an aggregate thereof, or a gene encoding same, is increased compared to a normal blood control sample.

In an embodiment of the present disclosure, the step of measuring protein levels is performed using one or more agents selected from the group consisting of an antibody, an aptamer, a ligand, a peptide nucleic acid(PNA), and an oligopeptide. Specifically, for example, expressions of GLUT3, USP14, α-synuclein, or AIMP2 proteins in the blood sample of a diagnostic target and in a normal blood control sample may be measured and compared by using an antibody specifically binding to GLUT3, USP14, α-synuclein, or AIMP2 proteins of the present disclosure. The method involves allowing the antibody to form an antigen-antibody complex with GLUT3, USP14, α-synuclein, or AIMP2 proteins in the biological sample and detecting same.

The term "antigen-antibody complex," as used herein, refers to a complex of a protein antigen present in a biological sample and an antibody recognizing same, for confirming the presence or absence of the corresponding gene. Detection of the antigen-antibody complex may be carried out by methods known in the art, such as spectroscopic, photochemical, biochemical, immunochemical, electrical, photo-absorptive, chemical methods, and other various methods.

In an embodiment of the present disclosure, the step of measuring gene expression levels is performed using one or more agents selected from the group consisting of primers, probes, and antisense nucleotides. Specifically, for example, measurement or comparison of the expression levels of genes encoding GLUT3, USP14, α-synuclein, or AIMP2 proteins of the present disclosure may be performed using reverse transcription polymerase chain reaction, competitive reverse transcription polymerase chain reaction, real-time reverse transcription polymerase chain reaction, RNase protection assay, Northern blotting, or DNA chips, but with no limitations thereto. Through such measurement methods, the amount of mRNA in a normal control group and the amount of mRNA in a subject to be diagnosed for disease occurrence can be confirmed, and by comparing the expression levels, it is possible to diagnose or predict the occurrence of Parkinson's disease.

The "method for providing information for diagnosing Parkinson's disease" of the present disclosure relates to a diagnostic method implemented using the "composition for diagnosing Parkinson's disease" of another aspect of the present disclosure described above, and overlapping content is incorporated by reference, with repetitive description omitted in order to avoid excessive complexity of the present specification.

Another aspect of the present disclosure provides a method for screening a therapeutic agent for Parkinson's disease, the method including the steps of:
a) administering a drug candidate to a Parkinson's disease animal model in which the expression level of GLUT3 protein, aggregates thereof, or genes encoding same in blood is increased;
b) collecting blood from the animal model after step a) and measuring the expression level of the protein or mRNA in the collected blood; and
c) determining the drug candidate as a therapeutic agent for Parkinson's disease when the expression level of GLUT3 protein or gene in the collected blood is significantly decreased by the administration of the drug candidate.

The administering in step a) of the present disclosure may be carried out via an oral or parenteral route, and a person skilled in the art may select an appropriate route of administration depending on the properties of the drug candidate.

In the method for screening a therapeutic agent for Parkinson's disease according to an embodiment of the present disclosure, step a) includes the following step a-1) and step c) includes the following step c-1):
a-1) administering a drug candidate to a Parkinson's disease animal model in which the expression level of GLUT3 and at least one protein selected from the group consisting of USP14, α-synuclein, and AIMP2, an aggregate thereof, or a gene encoding same in blood is increased; and
c-1) determining the drug candidate as a therapeutic agent for Parkinson's disease when the expression level of GLUT3, at least one protein selected from the group consisting of USP14, α-synuclein, and AIMP2, an aggregate thereof, or a gene encoding same, in the collected blood is significantly decreased by the administration of the drug candidate.

### Advantageous Effects of Invention

The features and advantages of the present disclosure can be summarized as follows:
(a) The present disclosure provides a composition for diagnosing Parkinson's disease.
(b) The present disclosure provides a kit for diagnosing Parkinson's disease.
(c) The present disclosure provides a method for providing information for diagnosing Parkinson's disease.
(d) The present disclosure provides a method for screening therapeutic agents for Parkinson's disease.
(e) By utilizing the composition and diagnostic kit for diagnosing Parkinson's disease of the present disclosure, Parkinson's disease can be diagnosed very rapidly and simply at an early stage through a blood test.

### Brief Description of Drawings

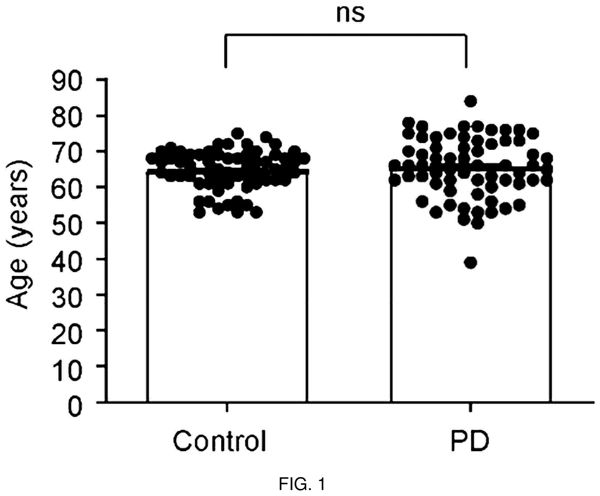

FIG. 1 shows the age distribution of Parkinson's disease patient groups and normal control groups (Parkinson's disease group n = 80, normal control group n = 78).

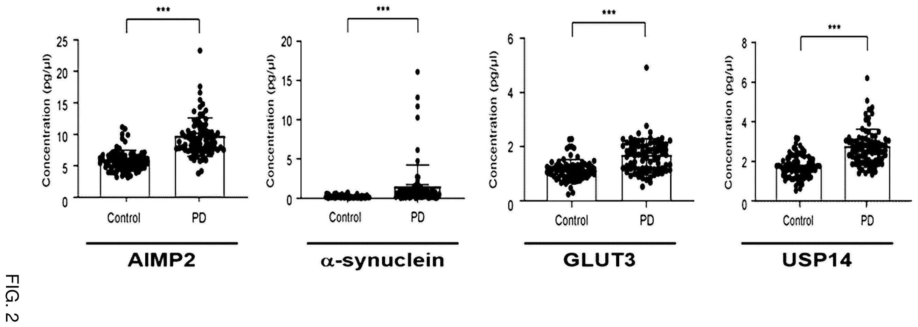

FIG. 2 is a quantitative analysis graph of protein markers(GLUT3, USP14, α-synuclein, and AIMP2) in plasma derived from Parkinson's disease patients and age-matched normal controls based on Sandwich ELISA(quantitative data are shown as mean ± standard error, significance determined by unpaired Student's t-test, **p < 0.01, ***p < 0.001).

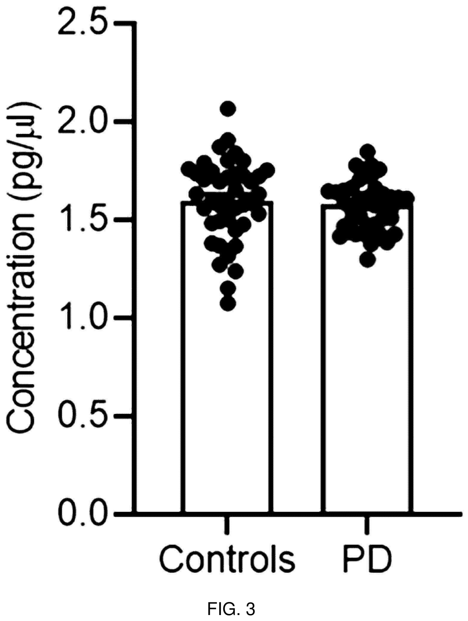

FIG. 3 is a quantitative analysis graph of the plasma protein marker DJ-1 in plasma derived from Parkinson's disease patients and age-matched normal controls based on Sandwich ELISA(quantitative data are shown as mean ± standard error, significance determined by unpaired Student's t-test, **p < 0.01, ***p < 0.001).

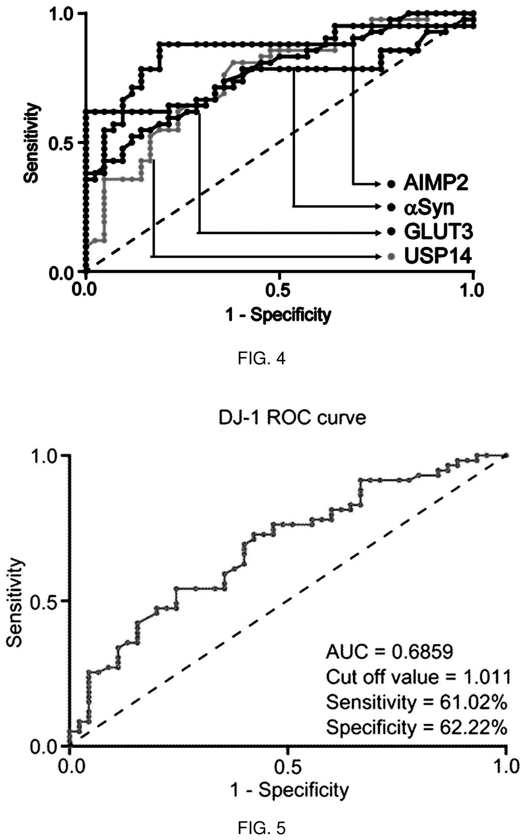

FIG. 4 shows the ROC curve analysis and diagnostic accuracy analysis results for plasma protein markers(GLUT3, USP14, α-synuclein, and AIMP2). It presents ROC curve analyses and sensitivity/1-specificity graphs for plasma concentration values of each protein marker in normal controls and Parkinson's disease patients.
   FIG. 5 shows the ROC curve analysis and diagnostic accuracy analysis results for the plasma protein marker DJ-1. It presents ROC curve analyses and sensitivity/1-specificity graphs for plasma concentration values of the protein marker in normal controls and Parkinson's disease patients.

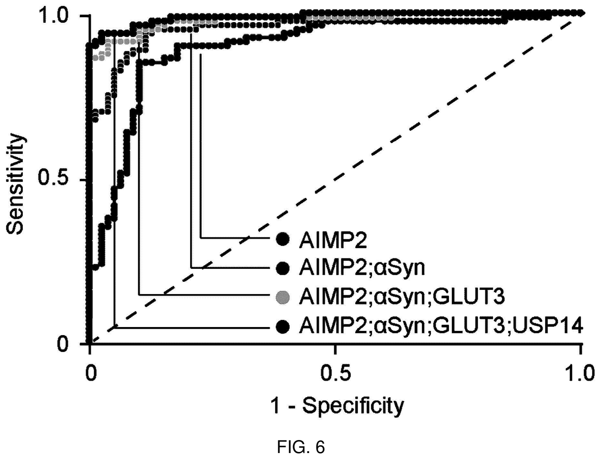

FIG. 6 shows the ROC curve analysis and diagnostic accuracy analysis results of a logistic regression-based multi-marker panel. Logistic regression functions were calculated for analyzing diagnostic accuracy of AIMP2 alone or in combination with α-synuclein, GLUT3, and USP14 markers, and ROC curves were analyzed based thereon.

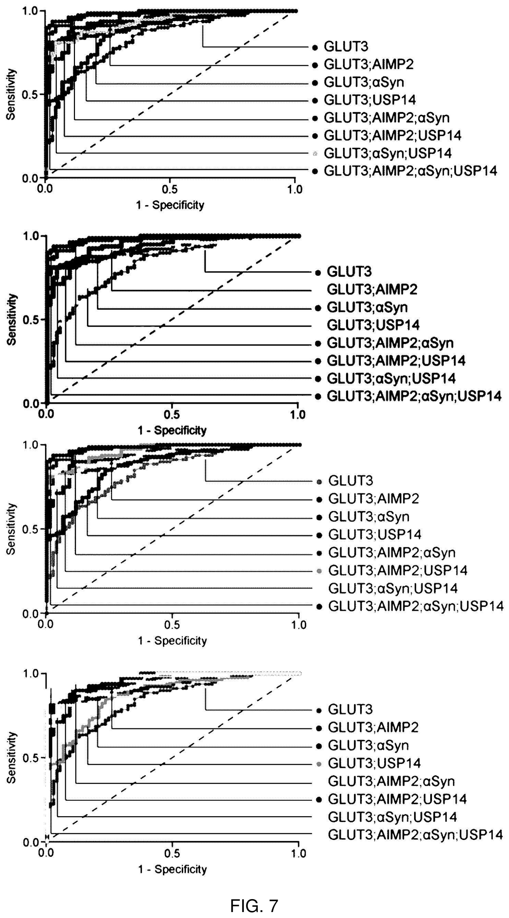

FIG. 7 shows the ROC curve analysis and diagnostic accuracy analysis results of a logistic regression-based multi-marker panel. Logistic regression functions were calculated for analyzing diagnostic accuracy of GLUT3 alone or in combination with α-synuclein, AIMP2, and USP14 markers, and ROC curves were analyzed based thereon.

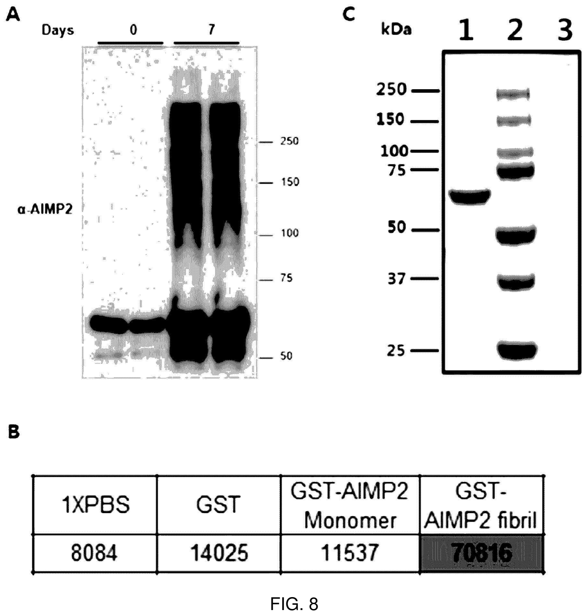

FIG. 8 illustrates the production and validation of an AIMP2 aggregate antigen:
   A: Western blot analysis results for GST-AIMP2 recombinant protein (0) and GST-AIMP2 aggregate obtained after a 7-day aggregation reaction (7); B: amyloid structure analysis of recombinant GST-AIMP2 monomer and GST-AIMP2 aggregate (GST-AIMP2 fibril) by Thioflavin T fluorescence analysis; and C: Coomassie blue staining results of the GST-AIMP2 aggregate antigen (1: BSA, 2: size marker, 3: GST-AIMP2 aggregate).
FIG. 9 shows the production of specific monoclonal antibody clones against AIMP2 aggregate conformations and the results of their isotype analysis:
   A: eight antibody-producing clones binding to AIMP2 monomer and/or AIMP2 aggregate; and B: isotype analysis results of the eight clones.
FIG. 10 shows the reactivity analysis results of AIMP2 antibody clones against the normal structure (monomer) and aggregate structure of AIMP2:
   A: dot blot analysis results of purified antibodies from the eight clones; and B: Western blot analysis results of purified antibodies from the eight clones.
FIG. 11 is a table comprehensively summarizing the three-dimensional structure-specific reactivity of the eight AIMP2 antibody clones.
FIG. 12 shows the sequence analysis results of antibodies produced from AIMP2 aggregate conformation-specific antibody clones (5B2 and 6C7):
   light green: CDH1, CDH2, and CDH3 amino acid sequences; blue: CDRL1, CDRL2, and CDRL3 amino acid sequences; gray: N-terminal amino acid sequences outside of those analyzed by Igblast; gray and underlined: invariant regions within the complete heavy and light chains of humanized chimeric antibodies; and red boxes: regions of sequence differences between the two clones.

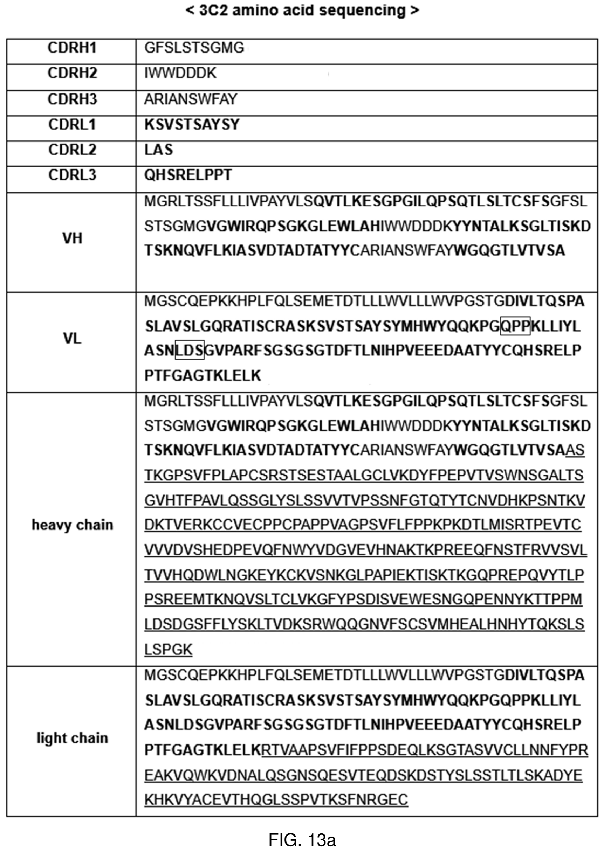

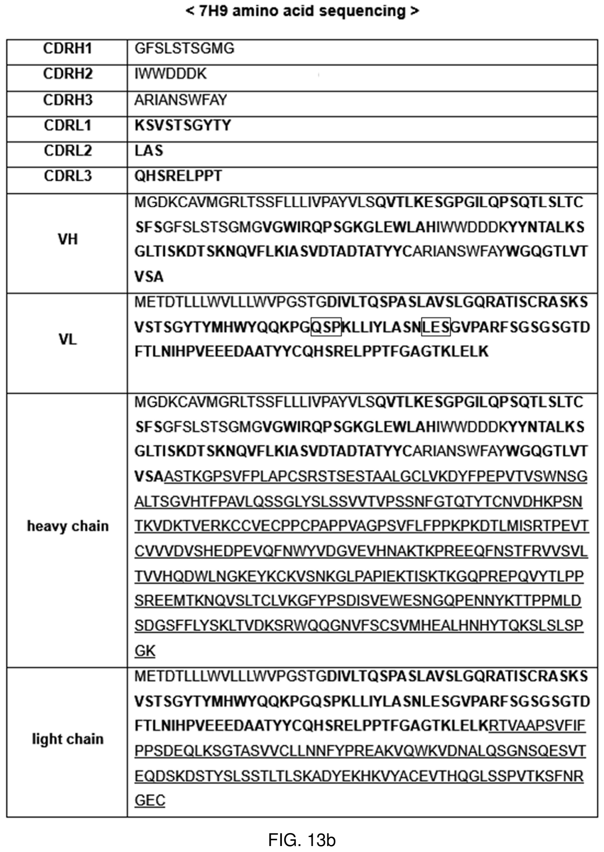

FIG. 13 shows the sequence analysis results of antibodies produced from AIMP2 monomer conformation-specific antibody clones (3C2 and 7H9):
   light green: CDH1, CDH2, and CDH3 amino acid sequences; blue: CDRL1, CDRL2, and CDRL3 amino acid sequences; gray: N-terminal amino acid sequences outside of those analyzed by Igblast; gray and underlined: invariant regions within the complete heavy and light chains of humanized chimeric antibodies; and red boxes: regions of sequence differences between the two clones.

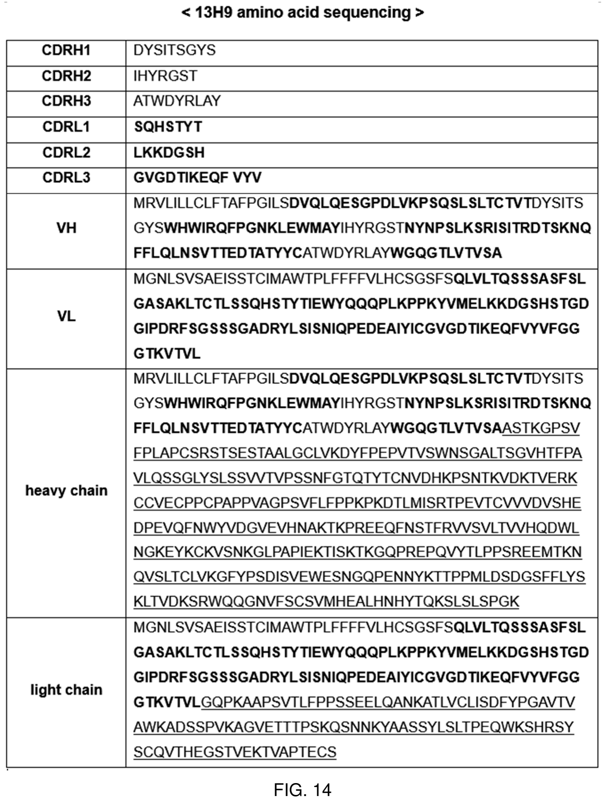

FIG. 14 shows the sequence analysis results of antibodies produced from an antibody clone (13H9) binding to both monomeric and aggregated conformations of AIMP2:
   light green: CDH1, CDH2, and CDH3 amino acid sequences; blue: CDRL1, CDRL2, and CDRL3 amino acid sequences; gray: N-terminal amino acid sequences outside of those analyzed by Igblast; and gray and underlined: invariant regions within the complete heavy and light chains of humanized chimeric antibodies.

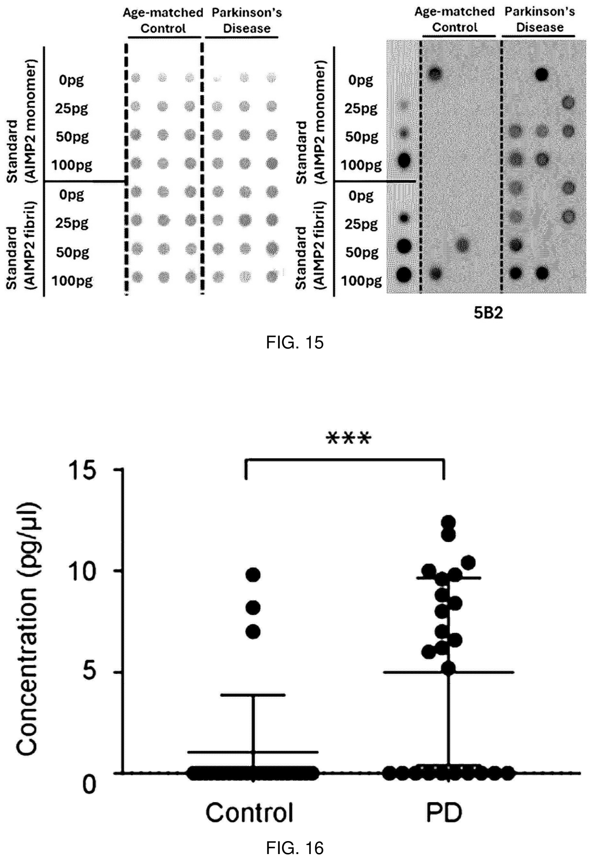

FIG. 15 shows images of ponceau staining to confirm the total protein amounts in plasma proteins of age-matched controls and Parkinson's disease patients bound to a nitrocellulose membrane, and dot blot results of the amounts of AIMP2 aggregates present in each plasma sample using the 5B2 antibody.
FIG. 16 is a graph of concentration values of AIMP2 aggregate protein present in each plasma sample, calculated based on the dot blot signal intensities of recombinant AIMP2 aggregate proteins from standards in FIG. 15 (n = 24 controls and 24 PD).

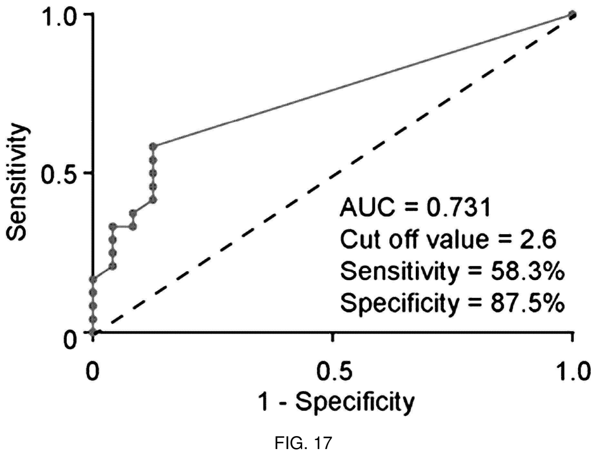

FIG. 17 is an ROC curve analysis for evaluating the validity of plasma AIMP2 aggregate concentration as a diagnostic marker for Parkinson's disease (n = 24 controls and 24 PD). AUC, cutoff values, and diagnostic sensitivity and specificity values are shown below the graph.

### Best Mode for Carrying out the Invention

A better understanding of the present disclosure may be obtained through the following examples that are set forth to illustrate, but are not to be constructed to limit, the scope of the present invention.

### EXAMPLES

### EXAMPLE 1: Preparation of Plasma Samples from Parkinson's Disease Patients and Control Group

Blood samples were obtained from 80 patients with Parkinson's disease and 78 age-matched normal controls at Hanyang University Hospital. Approval was obtained from the Institutional Review Board(IRB) of Hanyang University Hospital, and the approval number is HY-2021-06-040. As shown in FIG. 1 and Table 1 below, the average age was 64 years, with no significant age difference observed between the two groups.

**TABLE 1**

| Summary of Age and Sex Distribution of Normal Controls and Parkinson's Disease Patients | | |
|---|---|---|
| | Normal Controls | Parkinson's Patients |
| Age average | 64.15 | 64.93 |
| Sex | M = 36 | M = 41 |
| | F = 42 | F = 39 |

Blood samples collected in sodium heparin tubes(7 cc) were centrifuged at 3,000 rpm for 10 minutes at 4°C. One cc of plasma was transferred into each EP tube and stored at -70°C. The plasma was subsequently used for plasma protein marker analysis.

### EXAMPLE 2: Sandwich ELISA (Enzyme-Linked Immunosorbent Assay) Analysis and Absolute Quantification

AIMP2(aminoacyl-tRNA synthetase complex interacting multifunctional protein-2), USP14(ubiquitin-specific protease 14), GLUT3(glucose transporter 3), DJ-1, and α-synuclein, which served as candidate marker proteins for the diagnosis of Parkinson's disease, were quantified using Sandwich ELISA.

Capture antibodies were diluted in coating buffer at 1:1000 to 1:2000 to prepare a coating solution. All buffers, including the coating buffer, were from an ELISA buffer kit (CNB0011, Invitrogen). The coating solution was applied at 100 µL per well to a 96-well plate. The plate was covered and incubated overnight (12 to 18 hours) at 2 to 8°C. After incubation, the solution in each well was aspirated, and each well was washed once with 200 µL of wash buffer. After washing, the plate was inverted and tapped on absorbent paper to remove residual liquid. Blocking was then performed with 200 µL of blocking buffer per well for 1 hour at room temperature. After 1 hour, the blocking buffer was aspirated, and excess liquid was removed. Standard samples and plasma samples were diluted with blocking buffer to a final volume of 100 µL and prepared in advance. 100 µL of the standard and plasma samples were added to the designated wells. The plate was incubated at room temperature for 1 hour with shaking (~500 rpm). After 1 hour, each well was aspirated and washed five times with 200 µL of wash buffer. The plate was then inverted and dried thoroughly. Detection antibody solutions were prepared by diluting detection antibodies 1:1000 to 1:2000 in blocking buffer. 100 µL of the detection antibody solution was added to each well. The plate was incubated at room temperature for 2 hours with shaking (~500 rpm). After 2 hours, each well was aspirated and washed five times with 200 µL of wash buffer. The plate was inverted to remove residual liquid. A Streptavidin-HRP solution was prepared by diluting 1:5000 in blocking buffer. 100 µL of the Streptavidin-HRP solution was added to each well. The plate was incubated at room temperature for 30 minutes with shaking (~500 rpm). After 30 minutes, each well was aspirated and washed five times with 200 µL of wash buffer. The plate was inverted and dried thoroughly. 100 µL of TMB substrate solution was then added to each well and incubated at room temperature for 30 minutes. After incubation, 100 µL of stop solution was added to each well. Absorbance was measured at 450 nm within 30 minutes after adding the stop solution. Results were calculated using log-log or 4-parameter curve fitting.

The results are shown in FIGS. 2 and 3 and Table 2 below.

**TABLE 2**

| Mean Concentration and Standard Error of Plasma Protein Markers in Plasma Derived from Normal Controls and Parkinson's Disease Patients Based on ELISA Analysis | | |
|---|---|---|
| Marker | Plasma Concentration (pg/µL) | |
| | Normal Group | Parkinson's disease patients |
| GLUT3 | 1.65 ± 0.06 | 2.63 ± 0.10 |
| USP14 | 1.09 ± 0.04 | 1.61 ± 0.07 |
| α-synuclein | 0.22 ± 0.02 | 1.44 ± 0.32 |
| AIMP2 | 5.54 ± 0.19 | 9.34 ± 0.36 |
| DJ-1 | 1.59 ± 0.21 | 1.57 ± 0.13 |

As shown in FIGS. 2 and 3, the expression levels of four proteins (GLUT3, USP14, α-synuclein, and AIMP2), excluding DJ-1, were found to be significantly increased in the blood of Parkinson's disease patients.

Table 2 shows the mean plasma concentrations of four proteins (GLUT3, USP14, α-synuclein, and AIMP2) in age-matched normal controls and Parkinson's disease patients. The mean plasma concentration of GLUT3 was measured at 1.68 pg/µL in the normal group and 2.49 pg/µL in the Parkinson's disease group(a 48% increase). The mean plasma concentration of USP14 was measured at 1.17 pg/µL in the normal group and 1.66 pg/µL in the Parkinson's disease group(a 41% increase). The mean plasma concentration of α-synuclein was measured at 0.14 pg/µL in the normal group and 1.92 pg/µL in the Parkinson's disease group(a 13-fold increase). The mean plasma concentration of AIMP2 was measured at 6.38 pg/µL in the normal group and 10.44 pg/µL in the Parkinson's disease group(a 63% increase).

### EXAMPLE 3: Analysis of Diagnostic Sensitivity and Specificity of Individual Markers via Receiver Operating Characteristic(ROC) Curve Analysis

To evaluate whether individual markers can distinguish normal controls from Parkinson's disease patients based on the plasma concentrations of protein markers(FIGS. 2 and 3) in the Parkinson's patient group and normal control, ROC curve analysis was performed.

The O.D. values obtained from the ELISA analysis of Example 2 were normalized. Thereafter, using GraphPad Prism program, O.D. values for controls or actual concentration values were entered in column A, and O.D. values for PD were entered in column B. After input, "Analyze" was clicked in the data table window and "Receiver-operator characteristic curve" was selected. In the ROC dialog, the columns containing the control and Parkinson's disease results were designated, the output was set to "fraction" or "percentage," and the analysis was run. As analysis results, the area under the curve (AUC), sensitivity, specificity, and the ROC curve were obtained. Cutoff values were obtained as a table of ROC curve coordinates from Prism; three portions-cutoff value, sensitivity, and specificity-were exported to Excel. Sensitivity and specificity were plotted as a line chart, and the numerical value at the intersection of the two graphs was interpreted as the cutoff value, with the sensitivity and specificity at that point interpreted as the sensitivity and specificity for the ROC curve.

The results are shown in FIGS. 4 and 5 and Table 3.

**TABLE 3**

| Summary of AUC Values by Individual Markers from ROC Curve Analysis and Sensitivity/Specificity at the Diagnostic Cutoff Value | | | | |
|---|---|---|---|---|
| Marker | AUC | Cut off value (pg/µl) | Sensitivity (%) | Specificity (%) |
| GLUT3 | 0.7713 | 1.907 | 66.67 | 71.43 |
| USP14 | 0.7551 | 1.195 | 66.70 | 66.70 |
| α-Synuclein | 0.7577 | 0.1750 | 66.67 | 66.67 |
| AIMP2 | 0.8549 | 7.460 | 78.57 | 80.95 |
| DJ-1 | 0.6859 | 1.011 | 61.02 | 62.22 |

As shown in FIGS. 4 and 5 and Table 3 above, the individual markers exhibited relatively high AUC values, with AIMP2 showing the best AUC (0.8549). The cutoff values that maximized sensitivity and specificity were identified; α-synuclein, GLUT3, and USP14 exhibited sensitivities at the 66% level. DJ-1 showed specificity at the 62% level, α-synuclein and USP14 at the 66% level, whereas GLUT3 showed a relatively high specificity of 71%. AIMP2, which had the best AUC value, showed a sensitivity of 78.57% and a specificity of 80.95% at a cutoff value of 7.46 ng/µL.

### EXAMPLE 4: Multimarker Logistic Regression Analysis and ROC Curve Analysis by Multimarker Panel (1)

A multimarker panel was constructed by combining AIMP2 and α-synuclein, which are Parkinson's disease diagnostic markers, with GLUT3 and USP14 newly identified by the inventors, and logistic regression analysis was conducted to verify whether use of the constructed multimarker panel could improve diagnostic accuracy for Parkinson's disease.

For each antibody, the normalized O.D. values or actual concentration values obtained via the ELISA of Example 2 were arranged in a single row for the control group and the Parkinson's disease group in Excel, and another column was entered as "control = 0, Parkinson's disease = 1." The SPSS program was run and the data was pasted into the Data View window. In the Variable View window, the meaning of each column was entered, and for the column entered as "control = 0, Parkinson's disease = 1," value labels "0 = control, 1 = Parkinson's disease" were added. Then "Analyze → Regression → Binary Logistic" was clicked to open the binary logistic window; the dependent variable was set to "control = 0, Parkinson's disease = 1," and the independent variables AIMP2, αSyn, GLUT3, and USP14 were moved into the block field. For "Method," "Enter" (enter all variables simultaneously) was selected. Upon clicking "Save," a dialog appeared to select predicted values, influence, and residuals; by default, probability, classification, Cook's distance, and standardized residuals were selected and "Continue" was clicked. After analysis completion, the selected items were confirmed to be saved in the Data View window. "Probability (P)" stores the predicted event probability for each case. "Group (G)" indicates the predicted group assignment for each case based on predicted probability. "Standardized (N)" residuals are the standardized values obtained by dividing the residuals of each observation by the standard error of the residuals and serve as an important criterion for identifying outliers. "Cook's distance" identifies influential observations. Next, "Options" was clicked to check four itemsclassification table, Hosmer-Lemeshow goodness of fit, iteration history, and confidence intervals for exp-and "Continue" was clicked. "OK" was clicked to review the analysis results. From the analysis results, AUC, sensitivity, specificity, and cutoff values were obtained from the ROC curve; additionally, the goodness of fit of the logistic regression model was obtained as a p-value from the Hosmer-Lemeshow table. The odds ratio (OR) was also calculated using OR = P/(1 - P). Finally, an estimated regression equation for NPD/PD classification was constructed from the logistic regression.

The log probability functions by marker panel from the logistic regression analysis are shown in Table 4 below.

**TABLE 4**

| Log Probability Functions for Each Marker Panel by Logistic Regression Analysis | |
|---|---|
| Panel | Log Probability Functions [In(P/1 - P)] |
| AIMP2 | -5.994 + 0.8567X_{AIMP2} |
| AIMP2, αSyn | -9.041 + 0.9499X_{AIMP2}+ 6.487X_{αsyn} |
| AIMP2, αSyn, GLUT3 | -15.46 + 1.019X_{AIMP2}+ 7.965X_{αsyn}+ 2.627X_{GLUT3} |
| AIMP2, αSyn, GLUT3, USP14 | -21.41 + 1.192X_{AIMP2}+ 9.811X_{αsyn}+ 2.940X_{GLUT3}+ 2.658X_{USP14} |

The results are shown in FIG. 6 and Table 5.

As shown in FIG. 6, optimal logistic regression functions were derived while increasing the number of combined markers - e.g., AIMP2 alone; AIMP2 + α-synuclein panel; AIMP2 + α-synuclein + GLUT3 panel; and AIMP2 + α-synuclein + GLUT3 + USP14 panel. ROC curve analysis was performed based on the logistic regression function derived from each multimarker panel.

**TABLE 5**

| Summary of AUC, Cut-off Probability, and Diagnostic Sensitivity/Specificity of ROC Curves Based on Logistic Regression by Multimarker Panel | | | | |
|---|---|---|---|---|
| Panel | AUC | Cut off (Probability) | Sensitivity (%) | Specificity (%) |
| AIMP2 | 0.8974 | 0.4805 | 85.00 | 87.18 |
| AIMP2, αSyn | 0.9603 | 0.4273 | 88.75 | 89.74 |
| AIMP2, αSyn, GLUT3 | 0.9798 | 0.4536 | 91.25 | 92.31 |
| AIMP2, αSyn, GLUT3, USP14 | 0.9878 | 0.4685 | 93.75 | 94.87 |

Consequently, as shown in FIG. 6 and Table 5 above, the AUC increased in all multimarker panels compared with AIMP2 alone, with the highest AUC of 0.9878 observed in the panel combining all four markers. Using the probability that yielded maximal sensitivity and specificity as the cutoff value, analysis of sensitivity and specificity for each panel revealed that, whereas AIMP2 alone showed a sensitivity of 85% and a specificity of 87.18%, the four-marker panel showed a sensitivity of 93.75% and a specificity of 94.87% at a cutoff of 0.4685, indicating a remarkable improvement in diagnostic accuracy for Parkinson's disease.

### EXAMPLE 5: Multimarker Logistic Regression Analysis and ROC Curve Analysis by Multimarker Panel (2)

A multimarker panel was constructed by combining GLUT3 and USP14 newly identified by the present inventors with the Parkinson's disease diagnostic markers AIMP2 and α-synuclein, and logistic regression analysis was performed to verify whether diagnostic accuracy for Parkinson's disease could be improved using the constructed multimarker panel. The analysis was performed in the same manner as the logistic regression method of Example 4. The log probability functions for each marker panel by logistic regression analysis are shown in Table 6 below.

**TABLE 6**

| Log Probability Functions for Each Marker Panel by Logistic Regression Analysis | |
|---|---|
| Panel | Log Function [In(P/1-P)] |
| GLUT3 | -4.416 + 2.151X_{GLUT3} |
| GLUT3, AIMP2 | -11.30 + 2.500X_{GLUT3}+ 0.8427X_{AIMP2} |
| GLUT3, αSyn | -6.123 + 2.152X_{GLUT3}+ 4.256X_{αsyn} |
| GLUT3, USP14 | -6.790 + 1.999X_{GLUT3}+ 2.083X_{USP14} |
| GLUT3, AIMP2, αSyn | -15.46 + 2.627X_{GLUT3}+ 1.019X_{AIMP2}+ 7.965X_{αsyn} |
| GLUT3, AIMP2, USP14 | -14.17 + 2.469X_{GLUT3}+ 0.8595X_{AIMP2}+ 2.152X_{USP14} |
| GLUT3, αSyn, USP14 | -8.380 + 1.995X_{GLUT3}+ 4.363X_{αsyn}+ 1.999X_{USP14} |
| GLUT3, AIMP2, αSyn, USP14 | -21.41 + 2.940X_{GLUT3}+ 1.192X_{AIMP2}+ 9.811X_{αsyn}+ 2.658X_{USP14} |

The results are shown in FIG. 7 and Table 7.

As shown in FIG. 7, optimal logistic regression functions were derived while increasing the number of combined markers-GLUT3 alone; GLUT3 + AIMP2 panel; GLUT3 + α-synuclein panel; GLUT3 + USP14 panel; GLUT3 + AIMP2 + α-synuclein panel; GLUT3 + AIMP2 + USP14 panel; GLUT3 + α-synuclein + USP14 panel; and GLUT3 + AIMP2 + α-synuclein + USP14 panel. ROC curve analysis was performed based on the logistic regression function derived from each multimarker panel.

**TABLE 7 Summary of AUC, Cutoff Probability, and Diagnostic Sensitivity/Specificity of ROC Curves Based on Logistic Regression and Log Probability Function by Multimarker Panel**

| Panel | AUC | Cut off (Probability) | Sensitivity (%) | Specificity (%) |
|---|---|---|---|---|
| GLUT3 | 0.8375 | 0.3417 | 75.32 | 72.84 |
| GLUT3, AIMP2 | 0.9482 | 0.6414 | 88.89 | 89.61 |
| GLUT3, αSyn | 0.9170 | 0.5567 | 88.16 | 84.15 |
| GLUT3, USP14 | 0.8787 | 0.4381 | 79.49 | 77.50 |
| GLUT3, AIMP2, αSyn | 0.9798 | 0.7909 | 93.59 | 91.25 |
| GLUT3, AIMP2, USP14 | 0.9619 | 0.6813 | 87.34 | 86.08 |
| GLUT3, αSyn, USP14 | 0.9369 | 0.6126 | 87.18 | 85.00 |
| GLUT3, AIMP2, αSyn,USP14 | 0.9878 | 0.8382 | 96.15 | 93.75 |

Consequently, as shown in Table 7, the AUC increased in all multimarker panels compared with GLUT3 alone, with the highest AUC of 0.9878 observed in the panel combining all four markers. Using the probability that yielded maximal sensitivity and specificity as the cutoff value, analysis of each panel revealed that, whereas GLUT3 alone showed a sensitivity of 75.32% and a specificity of 72.84%, the four-marker panel showed a sensitivity of 96.15% and a specificity of 93.75% at a cutoff of 0.8382, indicating a marked increase in diagnostic accuracy for Parkinson's disease.

### EXAMPLE 6: Identification of Antibodies Specifically Binding to AIMP2 Monomer and/or AIMP2 Aggregates

### 6-1. Preparation and Verification of AIMP2 Aggregates

To generate monoclonal antibody clones labeling AIMP2 aggregates, recombinant AIMP2 aggregates were produced as antigens. A vector encoding GST-AIMP2 recombinant protein was transformed into BL21 cells, cultured, eluted using GST beads, and purified to obtain GST-AIMP2 recombinant protein. The purified GST-AIMP2 recombinant protein (GST-AIMP2 monomer) was incubated in a test tube at 37 °C and 250 rpm for 7 days to form high-molecular-weight aggregates (GST-AIMP2 fibrils). As a note, AIMP2 (monomer) without GST and AIMP2 aggregates (AIMP2 fibrils) were obtained by dialyzing the GST-AIMP2 and GST-AIMP2 aggregates, then treating with 100 µL Glutathione Sepharose 4B (GE Healthcare #17-0756-01) and 10 µL PreScission protease (GE Healthcare #27-0843-01), followed by overnight incubation. GST-AIMP2 and GST-AIMP2 aggregates were analyzed by Western blotting using an AIMP2-specific antibody (Proteintech Group, rabbit antibody), and the GST-AIMP2 aggregates were subjected to SDS-PAGE followed by Coomassie blue staining. As a result, GST-AIMP2 was detected and formation of GST-AIMP2 aggregates (Day 7) was confirmed (FIG. 8A). In addition, the amyloid structure of GST-AIMP2 aggregates was verified by Thioflavin T fluorescence analysis (FIG. 8B).

### 6-2. Generation of Hybridoma Cell Lines and Antibody Analysis

Using the GST-AIMP2 aggregate of FIG. 8C, which formed aggregates with an amyloid structure as in Example 6-1, as an antigen, ELISA analysis was performed to select hybridoma clones that did not react to GST but bound to GST-AIMP2 monomer (normal structure) and/or GST-AIMP2 aggregates (fibrils).

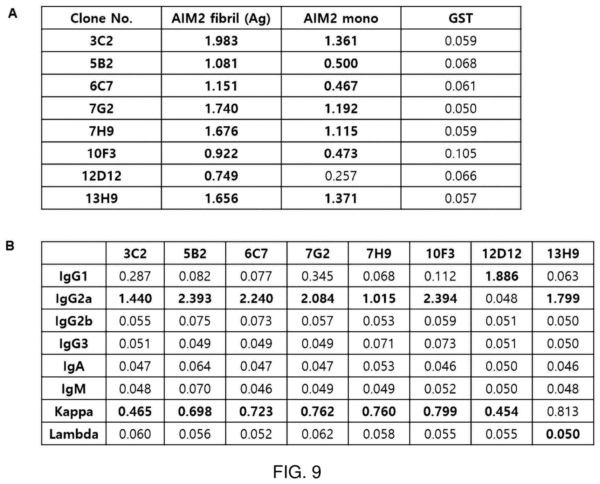

Eight hybridoma clones - 3C2, 5B2, 6C7, 7G2, 10F3, 12D12, and 13H9 - were selected (FIG. 9A). The culture supernatants of each hybridoma clone were collected, treated with Protein G IgG binding buffer (Thermo Fisher Scientific), and antibodies produced by each clone were purified using a Capturem Protein G Maxiprep Column (Cat. No. 635727). The isotypes of the purified antibodies from each clone were then analyzed (FIG. 9B).

### 6-3. Analysis of Conformation-Specific Reactivity to AIMP2 by Clone

### 6-3-1. Dot Blot Analysis

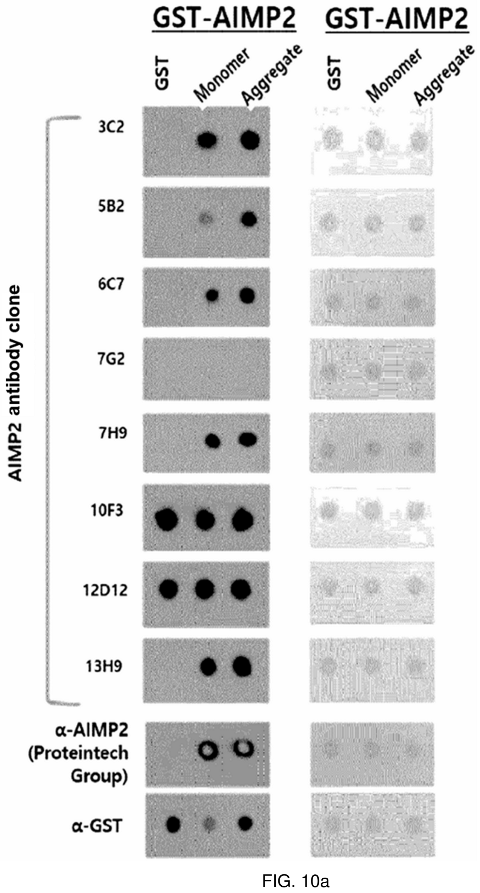

To verify the binding ability of each antibody produced by the eight selected clones to the three-dimensional structures of AIMP2, dot blot analysis was performed. Specifically, GST, GST-AIMP2 (monomer), and GST-AIMP2 aggregates (aggregate) were each diluted to the same concentration of 0.1 mg/mL with 1_{×} PBS, printed onto a nitrocellulose membrane, drawn through under vacuum, and loading was confirmed by ponceau staining, which was then removed; the membrane was blocked with 5% skim milk. The blocked membrane was washed three times with TBS-T, and the purified antibodies from each clone were used as primary antibodies and incubated at 4°C for 1 hour (Proteintech Group rabbit AIMP2 antibody and a GST antibody were used as controls). The membrane was washed three times with TBS-T and incubated with a secondary antibody (1:5000) for 30 minutes at room temperature. The blot was then developed using ECL. As a result, unlike the ELISA results, clones 10F3 and 12D12 also bound to GST and were therefore not suitable as AIMP2 antibody clones. In addition, the control rabbit AIMP2 antibody from Proteintech Group labeled both normal and aggregate structures of AIMP2 without selectivity; the 7G2 clone showed no binding to either AIMP2 monomer (normal structure) or aggregates; and the 10F3 and 12D12 clones recognized GST (FIG. 10A). Furthermore, the 5B2 and 6C7 antibody clones bound relatively well to AIMP2 aggregates, while the 3C2, 7H9, and 13H9 antibody clones showed similar levels of binding signals to both the normal and aggregate structures of AIMP2 (FIG. 10A).

### 6-3-2. Western Blot Analysis

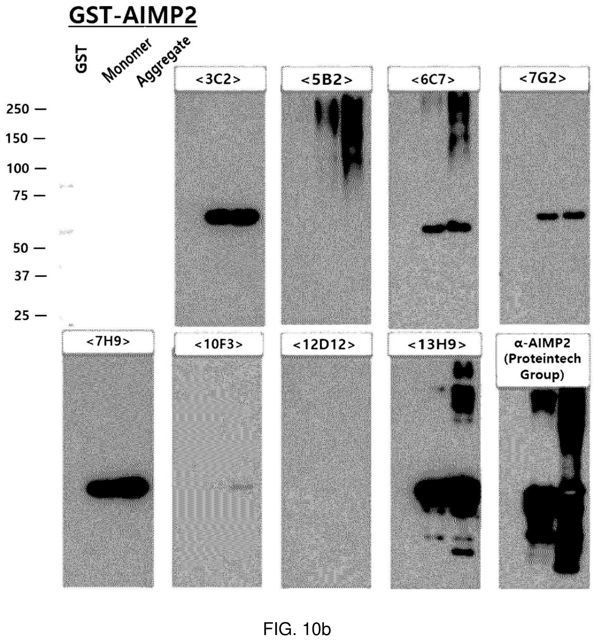

To verify antibody recognition of denatured proteins, GST, GST-AIMP2 (monomer), and GST-AIMP2 aggregates (aggregate) were separated by size on SDS-PAGE for Western blot analysis, and selective reactivity was analyzed using antibodies purified from each clone. As with the dot blot results of Example 6-3-1, the conventional Proteintech Group antibody detected not only the ~60 kDa GST-AIMP2 monomer (normal structure) but also high-molecular-weight GST-AIMP2 aggregates. In contrast, clones 5B2 and 6C7, which showed selectivity for AIMP2 aggregates in the dot blot analysis, selectively labeled high-molecular-weight AIMP2 aggregates; in particular, the 5B2 clone exhibited higher selectivity for AIMP2 aggregates than the 6C7 clone (FIG. 10B). In addition, clones 3C2 and 7H9 produced signals only for AIMP2 of normal structure (60 kDa), indicating that these clones are selective for the monomeric (normal) AIMP2 rather than aggregates (FIG. 10B). The antibody purified from clone 13H9 labeled AIMP2 regardless of conformation (FIG. 10B).

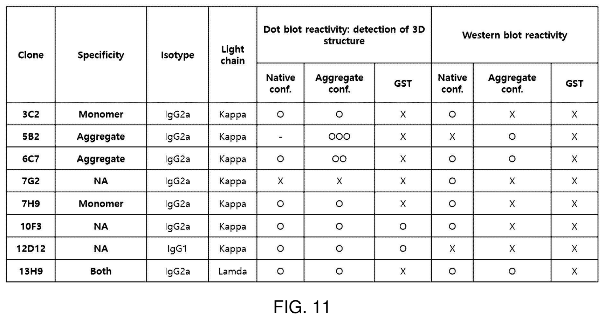

Collectively, the dot blot and Western blot results indicated that clones 10F3 and 12D12 also bound to GST, and clone 7G2 showed low binding to AIMP2, and thus these were not suitable as AIMP2 antibody clones. In contrast, clones 3C2 and 7H9 were classified as monoclonal antibody-producing clones specific for the normal structure of AIMP2; clones 5B2 and 6C7 were classified as monoclonal antibody-producing clones specific for the aggregate structure of AIMP2; and clone 13H9 was classified as a monoclonal antibody-producing clone non-specific to the AIMP2 conformation (FIG. 11).

### 6-4. Sequencing Analysis of CDS Amino Acid of AIMP2 Antibodies

To obtain the cDNA sequences encoding each antibody of the clones, the variable region sequences of the antibodies were obtained with reference to a previous report (Lena Meyer et al., A simplified workflow for monoclonal antibody sequencing, PLoS One, 2019). Specifically, for cDNA extracted from each hybridoma cell, the variable regions of the light and heavy chains were amplified by PCR using the primers listed in Table 8 below and cloned into a T-vector. The T-vectors containing the light-chain and heavy-chain variable regions were sequenced to obtain cDNA sequences, and the variable-region sequences were analyzed using IgBlast (IgBlast: https://www.ncbi.nlm.nih.gov/igblast/).

The amino acid sequences of the complementarity-determining regions(CDRs) were also analyzed.

**TABLE 8**

| Primer Name | Forward or reverse | Primer Sequence |
|---|---|---|
| ISPCR | Universal F primer | 5' aagcagtggtatcaacgcagag 3' |
| mOGK PCR | R primer for kappa chain | 5' acattgatgtctttggggtagaag 3' |
| mIGL PCR | R primer for lamda chain | 5' atcgtacacaccagtgtggc 3' |
| mOGHG PCR | R primer for heavy chain | 5' gggatccagagttccaggtc 3' |

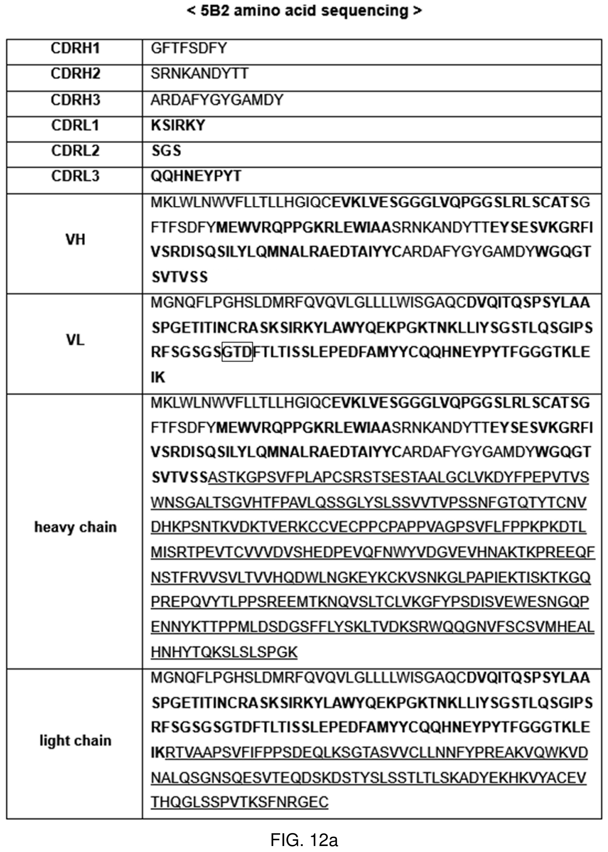

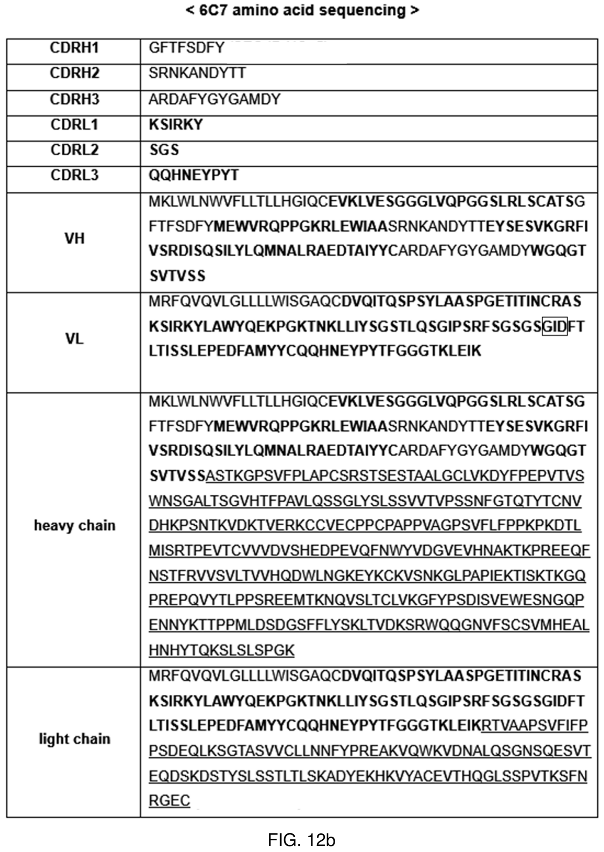

As a result, analysis of the cDNA sequences of clones 5B2 and 6C7, which showed specific reactivity to AIMP2 aggregates, confirmed identical amino acid sequences in their CDR regions, with only one amino acid difference among those coding for the light chain (FIG. 12). Accordingly, complete antibody sequences including the constant regions for generating humanized IgG were also constructed (FIG. 12). By the same method, analysis of the variable-region sequences of antibodies from clones 3C2 and 7H9, which exhibited specific binding to normal AIMP2, showed that the two clones also shared identical CDR amino acid sequences, with differences found only at two amino acid residues within the variable region of the light chain (FIG. 13). Likewise, complete sequences of humanized antibodies polymerized with human IgG constant regions were constructed (FIG. 13). In addition, the antibody clone 13H9, which detects both the normal and aggregate conformations of AIMP2, exhibited an antibody amino acid sequence distinct from the other four clones above (FIG. 14). The amino acid sequences of the variable regions, CDRs, and the complete sequences of the humanized antibodies are shown in FIG. 14.

### EXAMPLE 7: Analysis of AIMP2 Aggregate Expression in Serum of Controls and Parkinson's Disease Patients

To analyze expression of AIMP2 aggregates in the sera of controls and Parkinson's disease patients, dot blot and ROC curve analyses were performed.

An NC membrane was prepared by soaking in TBS buffer, set in a Bio-Dot microfiltration apparatus (Bio-Rad), and connected to a vacuum tube. After loading samples into each well, the apparatus was disassembled and the NC membrane was removed and washed three times for 5 minutes each in TBS-T buffer (TBS buffer containing 5% Tween 20). The NC membrane was blocked with 2.5% skim milk (in TBS-T) for 1 hour. The membrane was incubated with a primary antibody (overnight). After washing three times for 10 minutes each with TBS-T buffer, the membrane was incubated with an HRP-conjugated secondary antibody corresponding to the primary antibody for 1 hour 30 minutes. The membrane was then washed three times for 10 minutes each with TBS-T buffer and developed in a dark room using ECL solution. The quantitative values obtained from the dot blot were normalized, and ROC curve analysis was performed using GraphPad Prism.

The results are shown in FIGS. 15 to 17.

As shown in FIGS. 15 and 16, significantly higher expression levels of not only AIMP2 but also AIMP2 aggregates were observed in Parkinson's disease patients than in controls. As shown in FIG. 17, ROC curve analysis yielded an AUC value of 0.731, confirming that the concentration of AIMP2 aggregates can be used as a marker for diagnosing Parkinson's disease. The optimal cutoff value was 2.6 pg/µL, at which the sensitivity was 58.3% and the specificity was 87.5%.

The sequence listing used in the present disclosure is as shown in the table below.

**TABLE 9**

| SEQ.ID.NO | Sequence Name | Sequence |
|---|---|---|
| 1 | ISPCR - Universal F primer | 5' aagcagtggtatcaacgcagag 3' |
| 2 | mIGK PCR - R primer for kappa chain | 5' acattgatgtctttggggtagaag 3' |
| 3 | mIGL PCR - R primer for lamda chain | 5' atcgtacacaccagtgtggc 3' |
| 4 | mIGHG PCR - R primer for heavy chain | 5' gggatccagagttccaggtc 3' |
| 5 | 5B2/6C7-CDRH1 | GFTFSDFY |
| 6 | 5B2/6C7-CDRH2 | SRNKANDYTT |
| 7 | 5B2/6C7-CDRH3 | ARDAFYGYGA MDY |
| 8 | 5B2/6C7-CDRL1 | KSIRKY |
| 9 | 5B2/6C7-CDRL2 | SGS |
| 10 | 5B2/6C7-CDRL3 | QQHNEYPYT |
| 11 | 5B2/6C7-VH | MKLWLNWVFL LTLLHGIQCE VKLVESGGGL VQPGGSLRLS CATSGFTFSD FYMEWVRQPP GKRLEWIAAS RNKANDYTTE YSESVKGRFI VSRDISQSIL YLQMNALRAE DTAIYYCARD AFYGYGAMDY WGQGTSVTVS S |
| 12 | 5B2-VL | MGNQFLPGHS LDMRFQVQVL GLLLLWISGA QCDVQITQSP SYLAASPGET ITINCRASKS IRKYLAWYQE KPGKTNKLLI YSGSTLQSGI PSRFSGSGSG TDFTLTISSL EPEDFAMYYC QQHNEYPYTF GGGTKLEIK |
| 13 | 6C7-VL | MRFQVQVLGL LLLWISGAQC DVQITQSPSY LAASPGETIT INCRASKSIR KYLAWYQEKP GKTNKLLIYS GSTLQSGIPS RFSGSGSGID FTLTISSLEP EDFAMYYCQQ HNEYPYTFGG GTKLEIK |
| 14 | 5B2/6C7-Heavy chain | MKLWLNWVFL LTLLHGIQCE VKLVESGGGL VQPGGSLRLS CATSGFTFSD FYMEWVRQPP GKRLEWIAAS RNKANDYTTE YSESVKGRFI VSRDISQSIL YLQMNALRAE DTAIYYCARD AFYGYGAMDY WGQGTSVTVS SASTKGPSVF PLAPCSRSTS ESTAALGCLV KDYFPEPVTV SWNSGALTSG VHTFPAVLQS SGLYSLSSVV TVPSSNFGTQ TYTCNVDHKP SNTKVDKTVE RKCCVECPPC PAPPVAGPSV FLFPPKPKDT LMISRTPEVT CVVVDVSHED PEVQFNWYVD GVEVHNAKTK PREEQFNSTF RVVSVLTVVH QDWLNGKEYK CKVSNKGLPA PIEKTISKTK GQPREPQVYT LPPSREEMTK NQVSLTCLVK GFYPSDISVE WESNGQPENN YKTTPPMLDS DGSFFLYSKL TVDKSRWQQG NVFSCSVMHE ALHNHYTQKS LSLSPGK |
| 15 | 5B2-Light chain | MGNQFLPGHS LDMRFQVQVL GLLLLWISGA QCDVQITQSP SYLAASPGET ITINCRASKS IRKYLAWYQE KPGKTNKLLI YSGSTLQSGI PSRFSGSGSG TDFTLTISSL EPEDFAMYYC QQHNEYPYTF GGGTKLEIKR TVAAPSVFIF PPSDEQLKSG TASVVCLLNN FYPREAKVQW KVDNALQSGN SQESVTEQDS KDSTYSLSST LTLSKADYEK HKVYACEVTH QGLSSPVTKS FNRGEC |
| 16 | 6C7-Light chain | MRFQVQVLGL LLLWISGAQC DVQITQSPSY LAASPGETIT INCRASKSIR KYLAWYQEKP GKTNKLLIYS GSTLQSGIPS RFSGSGSGID FTLTISSLEP EDFAMYYCQQ HNEYPYTFGG GTKLEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 17 | 3C2/7H9-CDRH1 | GFSLSTSGMG |
| 18 | 3C2/7H9-CDRH2 | IWWDDDK |
| 19 | 3C2/7H9-CDRH3 | ARIANSWFAY |
| 20 | 3C2-CDRL1 | KSVSTSAYSY |
| 21 | 7H9-CDRL1 | KSVSTSGYTY |
| 22 | 3C2/7H9-CDRL2 | LAS |
| 23 | 3C2/7H9-CDRL3 | QHSRELPPT |
| 24 | 3C2-VH | MGRLTSSFLL LIVPAYVLSQ VTLKESGPGI LQPSQTLSLT CSFSGFSLST SGMGVGWIRQ PSGKGLEWLA HIWWDDDKYY NTALKSGLTI SKDTSKNQVF LKIASVDTAD TATYYCARIA NSWFAYWGQG TLVTVSA |
| 25 | 7H9-VH | MGDKCAVMGR LTSSFLLLIV PAYVLSQVTL KESGPGILQP SQTLSLTCSF SGFSLSTSGM GVGWIRQPSG KGLEWLAHIW WDDDKYYNTA LKSGLTISKD TSKNQVFLKI ASVDTADTAT YYCARIANSW FAYWGQGTLV TVSA |
| 26 | 3C2-VL | MGSCQEPKKH PLFQLSEMET DTLLLWVLLL WVPGSTGDIV LTQSPASLAV SLGQRATISC RASKSVSTSA YSYMHWYQQK PGQPPKLLIY LASNLDSGVP ARFSGSGSGT DFTLNIHPVE EEDAATYYCQ HSRELPPTFG AGTKLELK |
| 27 | 7H9-VL | METDTLLLWV LLLWVPGSTG DIVLTQSPAS LAVSLGQRAT ISCRASKSVS TSGYTYMHWY QQKPGQSPKL LIYLASNLES GVPARFSGSG SGTDFTLNIH PVEEEDAATY YCQHSRELPP TFGAGTKLEL K |
| 28 | 3C2-Heavy chain | MGRLTSSFLL LIVPAYVLSQ VTLKESGPGI LQPSQTLSLT CSFSGFSLST SGMGVGWIRQ PSGKGLEWLA HIWWDDDKYY NTALKSGLTI SKDTSKNQVF LKIASVDTAD TATYYCARIA NSWFAYWGQG TLVTVSAAST KGPSVFPLAP CSRSTSESTA ALGCLVKDYF PEPVTVSWNS GALTSGVHTF PAVLQSSGLY SLSSVVTVPS SNFGTQTYTC NVDHKPSNTK VDKTVERKCC VECPPCPAPP VAGPSVFLFP PKPKDTLMIS RTPEVTCVVV DVSHEDPEVQ FNWYVDGVEV HNAKTKPREE QFNSTFRVVS VLTVVHQDWL NGKEYKCKVS NKGLPAPIEK TISKTKGQPR EPQVYTLPPS REEMTKNQVS LTCLVKGFYP SDISVEWESN GQPENNYKTT PPMLDSDGSF FLYSKLTVDK SRWQQGNVFS CSVMHEALHN HYTQKSLSLS PGK |
| 29 | 7H9-Heavy chain | MGDKCAVMGR LTSSFLLLIV PAYVLSQVTL KESGPGILQP SQTLSLTCSF SGFSLSTSGM GVGWIRQPSG KGLEWLAHIW WDDDKYYNTA LKSGLTISKD TSKNQVFLKI ASVDTADTAT YYCARIANSW FAYWGQGTLV TVSAASTKGP SVFPLAPCSR STSESTAALG CLVKDYFPEP VTVSWNSGAL TSGVHTFPAV LQSSGLYSLS SVVTVPSSNF GTQTYTCNVD HKPSNTKVDK TVERKCCVEC PPCPAPPVAG PSVFLFPPKP KDTLMISRTP EVTCVVVDVS HEDPEVQFNW YVDGVEVHNA KTKPREEQFN STFRVVSVLT VVHQDWLNGK EYKCKVSNKG LPAPIEKTIS KTKGQPREPQ VYTLPPSREE MTKNQVSLTC LVKGFYPSDI SVEWESNGQP ENNYKTTPPM LDSDGSFFLY SKLTVDKSRW QQGNVFSCSV MHEALHNHYT QKSLSLSPGK |
| 30 | 3C2-Light chain | MGSCQEPKKH PLFQLSEMET DTLLLWVLLL WVPGSTGDIV LTQSPASLAV SLGQRATISC RASKSVSTSA YSYMHWYQQK PGQPPKLLIY LASNLDSGVP ARFSGSGSGT DFTLNIHPVE EEDAATYYCQ HSRELPPTFG AGTKLELKRT VAAPSVFIFP PSDEQLKSGT ASVVCLLNNF YPREAKVQWK VDNALQSGNS QESVTEQDSK DSTYSLSSTL TLSKADYEKH KVYACEVTHQ GLSSPVTKSF NRGEC |
| 31 | 7H9-Light chain | METDTLLLWV LLLWVPGSTG DIVLTQSPAS LAVSLGQRAT ISCRASKSVS TSGYTYMHWY QQKPGQSPKL LIYLASNLES GVPARFSGSG SGTDFTLNIH PVEEEDAATY YCQHSRELPP TFGAGTKLEL KRTVAAPSVF IFPPSDEQLK SGTASVVCLL NNFYPREAKV QWKVDNALQS GNSQESVTEQ DSKDSTYSLS STLTLSKADY EKHKVYACEV THQGLSSPVT KSFNRGEC |
| 32 | 13H9-CDRH1 | DYSITSGYS |
| 33 | 13H9-CDRH2 | IHYRGST |
| 34 | 13H9-CDRH3 | ATWDYRLAY |
| 35 | 13H9-CDRL1 | SQHSTYT |
| 36 | 13H9-CDRL2 | LKKDGSH |
| 37 | 13H9-CDRL3 | GVGDTIKEQF VYV |
| 38 | 13H9-VH | MRVLILLCLF TAFPGILSDV QLQESGPDLV KPSQSLSLTC TVTDYSITSG YSWHWIRQFP GNKLEWMAYI HYRGSTNYNP SLKSRISITR DTSKNQFFLQ LNSVTTEDTA TYYCATWDYR LAYWGQGTLV TVSA |
| 39 | 13H9-VL | MGNLSVSAEI SSTCIMAWTP LFFFFVLHCS GSFSQLVLTQ SSSASFSLGA SAKLTCTLSS QHSTYTIEWY QQQPLKPPKY VMELKKDGSH STGDGIPDRF SGSSSGADRY LSISNIQPED EAIYICGVGD TIKEQFVYVF GGGTKVTVL |
| 40 | 13H9-Heavy chain | MRVLILLCLF TAFPGILSDV QLQESGPDLV KPSQSLSLTC TVTDYSITSG YSWHWIRQFP GNKLEWMAYI HYRGSTNYNP SLKSRISITR DTSKNQFFLQ LNSVTTEDTA TYYCATWDYR LAYWGQGTLV TVSAASTKGP SVFPLAPCSR STSESTAALG CLVKDYFPEP VTVSWNSGAL TSGVHTFPAV LQSSGLYSLS SVVTVPSSNF GTQTYTCNVD HKPSNTKVDK TVERKCCVEC PPCPAPPVAG PSVFLFPPKP KDTLMISRTP EVTCVVVDVS HEDPEVQFNW YVDGVEVHNA KTKPREEQFN STFRVVSVLT VVHQDWLNGK EYKCKVSNKG LPAPIEKTIS KTKGQPREPQ VYTLPPSREE MTKNQVSLTC LVKGFYPSDI SVEWESNGQP ENNYKTTPPM LDSDGSFFLY SKLTVDKSRW QQGNVFSCSV MHEALHNHYT QKSLSLSPGK |
| 41 | 13H9-Light chain | MGNLSVSAEI SSTCIMAWTP LFFFFVLHCS GSFSQLVLTQ SSSASFSLGA SAKLTCTLSS QHSTYTIEWY QQQPLKPPKY VMELKKDGSH STGDGIPDRF SGSSSGADRY LSISNIQPED EAIYICGVGD TIKEQFVYVF GGGTKVTVLG QPKAAPSVTL FPPSSEELQA NKATLVCLIS DFYPGAVTVA WKADSSPVKA GVETTTPSKQ SNNKYAASSY LSLTPEQWKS HRSYSCQVTH EGSTVEKTVA PTECS |

## Claims

1. A composition for diagnosing Parkinson's disease, further comprising an agent for detecting at least one protein selected from the group consisting of GLUT3 (glucose transporter 3), USP14 (ubiquitin-specific protease 14), α-synuclein, and AIMP2 (aminoacyl-tRNA synthetase complex interacting multifunctional protein-2), an aggregate thereof, or a gene encoding same.

2. The composition for diagnosing Parkinson's disease, comprising an agent for detecting a GLUT3 protein, an aggregate thereof, or a gene encoding same.

3. The composition of claim 2, further comprising an agent for detecting at least one protein selected from the group consisting of USP14, α-synuclein, and AIMP2, an aggregate thereof, or a gene encoding same.

4. The composition of claim 1, wherein the protein is a protein present in a blood sample isolated from a human.

5. The composition of claim 1, wherein the agent for detecting the protein is at least one selected from the group consisting of an antibody, an aptamer, a ligand, a peptide nucleic acid (PNA), and an oligopeptide.

6. The composition of claim 1, wherein the agent for detecting the gene is at least one selected from the group consisting of primers, probes, and antisense nucleotides.

7. A kit for diagnosing Parkinson's disease, comprising the composition of any one of claims 1 to 6.

8. A method for providing information for diagnosing Parkinson's disease, the method comprising the steps of:
(a) measuring an expression level of GLUT3 protein, an aggregate thereof, or a gene encoding same in a blood sample isolated from a human; and
(b) providing information that the likelihood of Parkinson's disease is high when the expression level is increased compared to a normal blood control sample.

9. The method of claim 8, wherein step (a) includes the following step (a-1) and step (b) includes the following step (b-1):
(a-1) measuring an expression level of a combination of GLUT3 and at least one selected from the group consisting of USP14, α-synuclein, and AIMP2, an aggregate thereof, or a gene encoding same in a blood sample isolated from a human,
(b-1) providing information that the likelihood of Parkinson's disease is high when the expression level of a combination of GLUT3 and at least one selected from the group consisting of USP14, α-synuclein, and AIMP2, an aggregate thereof, or a gene encoding same in a blood sample isolated from a human are increased, compared to a normal blood control sample.

10. The method of claim 8, wherein the step of measuring protein levels is performed using at least one agent selected from the group consisting of an antibody, an aptamer, a ligand, a peptide nucleic acid (PNA), and an oligopeptide.

11. The method of claim 8, wherein the step of measuring gene expression levels is performed using at least one agent selected from the group consisting of primers, probes, and antisense nucleotides.

12. A method for screening a therapeutic agent for Parkinson's disease, the method comprising the steps of:
a) administering a drug candidate to a Parkinson's disease animal model in which the expression level of GLUT3 protein in blood, aggregates thereof, or a gene encoding the same is increased;
b) collecting blood from the animal model and measuring the expression level of the protein or mRNA in the collected blood after step a); and
c) determining the drug candidate as a therapeutic agent for Parkinson's disease when the expression level of GLUT3 protein, an aggregate thereof, or a gene encoding same in the collected blood shows a significant decrease due to administration of the drug candidate.

13. The method of claim 12, wherein step a) includes the following step a-1) and step c) includes the following step c-1):
a-1) administering a drug candidate to a Parkinson's disease animal model in which the expression level of a combination of GLUT3 and at least one protein selected from the group consisting of USP14, α-synuclein, and AIMP2, an aggregate thereof, or a gene encoding same in blood is increased; and
c-1) determining the drug candidate as a therapeutic agent for Parkinson's disease when the expression level in the collected blood of GLUT3, and at least one protein selected from the group consisting of USP14, α-synuclein, and AIMP2, an aggregate thereof, or a gene encoding same, shows a significant decrease due to administration of the drug candidate.
